(19) Europäisches Patentamt
European Patent Office
Office européen des brevets

(11) Veröffentlichungsnummer: **0 070 990**
**A2**

(12) **EUROPÄISCHE PATENTANMELDUNG**

(21) Anmeldenummer: 82104484.9

(22) Anmeldetag: 22.05.82

(51) Int. Cl.³: C 07 D 249/08
C 07 D 249/10, C 07 D 233/60
C 07 D 231/12, C 07 D 231/16
C 07 D 231/18, A 01 N 43/50
A 01 N 43/56, A 01 N 43/64

(30) Priorität: 04.06.81 DE 3122152
02.03.82 DE 3207483

(43) Veröffentlichungstag der Anmeldung:
09.02.83 Patentblatt 83/6

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Förster, Heinz, Dr.
Am Eckbusch 47
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert, Dr.
Im Waldwinkel 110
D-5060 Bergisch-Gladbach 2(DE)

(54) **Substituierte Phenoxybenzoesäure-azolylalkylester, Verfahren zu ihrer Herstellung und ihre Verwendung als Herbizide.**

(57) Substituierte Phenoxybenzoesäure-azolylalkylester der Formel

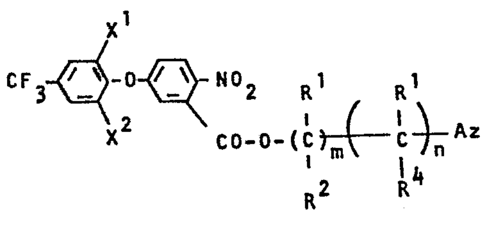

(I)

in welcher

$X^1$ und $X^2$ unabhängig voneinander für Wasserstoff oder Halogen stehen,

$R^1$, $R^2$, $R^3$ und $R^4$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salze, Metallsalz-Komplexe und quaternäre Alkyllierungsmittel-Additionssalze, mehrere Verfahren zu deren Herstellung und deren Verwendung als Herbizide.

Phenoxybenzoesäure-azolylalkylester der Formel

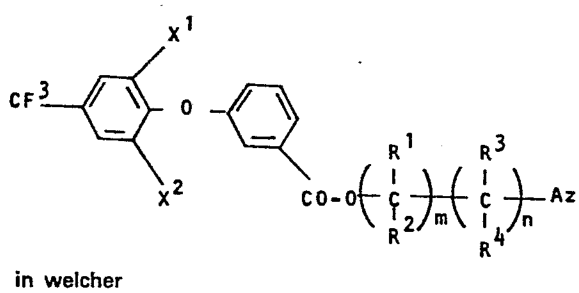

(IV)

in welcher

$X^1$, $X^2$, $R^1$, $R^2$, $R^3$, $R^4$, Az, m und n die oben angegebene Bedeutung haben,

und ein Verfahren zu deren Herstellung.

Halogensubstituierte Benzoesäurederivate der Formel

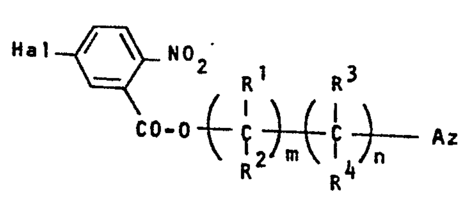

(VI)

./...

EP 0 070 990 A2

in welcher

$R^1$, $R^2$, $R^3$, $R^4$, Az m und n die oben angegebene Bedeutung haben und

Hal für Fluor, Chlor oder Brom steht,

und ein Verfahren zu deren Herstellung.

Phenoxy-benzoesäurechloride der Formel

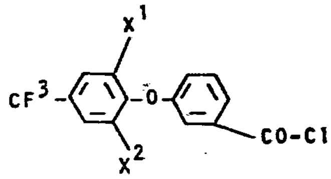

(V111)

in welcher

$X^1$ und $X^2$ die oben angegebene Bedeutung haben,

und ein Verfahren zu deren herstellung.

0070990

BAYER AKTIENGESELLSCHAFT 5090 Leverkusen, Bayerwerk

Zentralbereich
Patente, Marken und Lizenzen Dü-by-c
Ib

Substituierte Phenoxybenzoesäure-azolylalkylester, Verfahren zu ihrer Herstellung und ihre Verwendung als Herbizide

Die Erfindung betrifft neue substituierte Phenoxybenzoesäure-azolylalkylester, mehrere Verfahren zu deren Herstellung sowie deren Verwendung als Herbizide.

Es ist bereits bekannt geworden, daß bestimmte Phenoxybenzoesäure-Derivate herbizide Eigenschaften besitzen (vgl. DE-OS 2 311 638 und US-PS 4 063 929). So läßt sich zum Beispiel 5-(2-Chlor-4-trifluormethyl-phenoxy)-2-nitro-benzoesäure-Natriumsalz (Acifluorfen) zur Bekämpfung von Unkraut verwenden. Die herbizide Wirkung dieses Stoffes ist gut, jedoch ist die Selektivität nicht immer ausreichend.

Es wurden nun neue substituierte Phenoxybenzoesäure-azolylalkylester der Formel

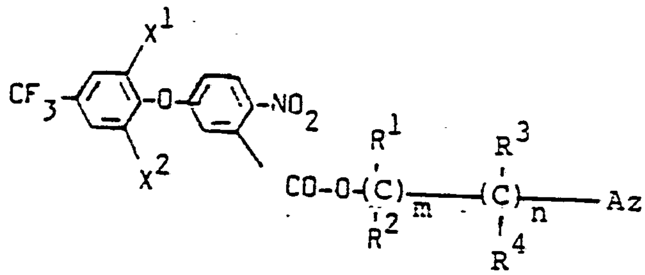

(I)

in welcher

$X^1$ und $X^2$ unabhängig voneinander für Wasserstoff oder Halogen stehen,

$R^1$, $R^2$, $R^3$ und $R^4$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salze, Metallsalz-Komplexe und quaternäre Alkylierungsmittel-Additionssalze gefunden.

Weiterhin wurde gefunden, daß man die neuen substituierten Phenoxybenzoesäure-azolylalkylester der Formel (I), deren Säureadditions-Salze, Metallsalz-Komplexe und quaternäre Alkylierungsmittel-Additionssalze erhält, wenn man

(a) substituierte Phenoxybenzoesäurechloride der Formel

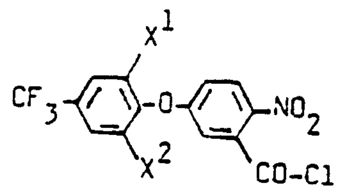

(II)

in welcher

$X^1$ und $X^2$ die oben angegebene Bedeutung haben,

mit Hydroxyalkyl-azolen der Formel

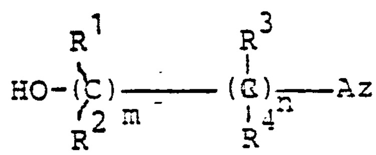

(III)

in welcher

$R^1$, $R^2$, $R^3$, $R^4$, Az, m und n die oben angegebene Bedeutung haben,

gegebenenfalls in Gegenwart eines Säureakzeptors und gegebenenfalls in Gegenwart eines Verdünnungsmittels umsetzt oder

(b) Phenoxybenzoesäure-azolylalkylester der Formel

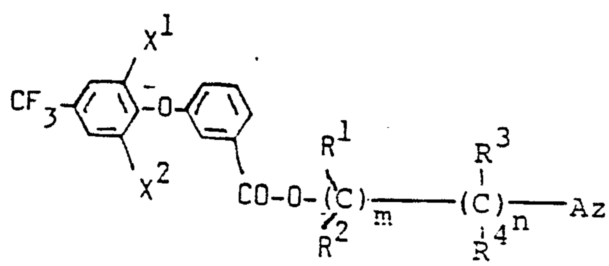

(IV)

in welcher

$X^1$, $X^2$, $R^1$, $R^2$, $R^3$, $R^4$, Az, m und n die oben angegebene Bedeutung haben,

mit Salpetersäure gegebenenfalls in Gegenwart eines Katalysators und gegebenenfalls in Gegenwart eines Verdünnungsmittels umsetzt, oder

(c) substituierte Phenole der Formel

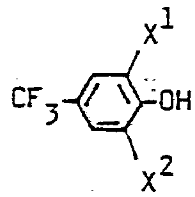

(V)

in welcher

$X^1$ und $X^2$ die oben angegebene Bedeutung haben,

mit halogen-substituierten Benzoesäurederivaten der Formel

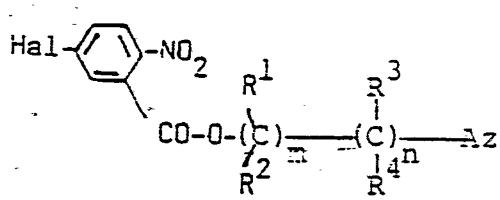

(VI)

in welcher

$R^1$, $R^2$, $R^3$, $R^4$, Az, m und n die oben angegebene Bedeutung haben und

Hal für Fluor, Chlor oder Brom steht,

gegebenenfalls in Gegenwart eines Säureakzeptors und gegebenenfalls in Gegenwart eines Verdünnungsmittels umsetzt,

und gegebenenfalls anschließend an die so erhaltenen substituierten Phenoxybenzoesäureazolylalkylester der Formel (I) eine Säure, ein Metallsalz oder ein Alkylierungsmittel addiert.

Schließlich wurde gefunden, daß sich die neuen substituierten Phenoxybenzoesäure-azolylalkylester der Formel (I), deren Säureadditions-Salze, Metallsalz-Komplexe und quaternäre Alkylierungsmittel-Additionssalze durch sehr gute herbizide Eigenschaften auszeichnen.

Überraschenderweise besitzen die erfindungsgemäßen substituierten Phenoxybenzoesäure-azolylalkylester der Formel (I) sowie deren Säureadditions-Salze, Metallsalz-Komplexe und quaternäre Alkylierungsmittel-Additionssalze eine wesentlich bessere selektive herbizide Wirksamkeit als das 5-(2-Chlor-4-trifluormethyl-phenoxy)-2-nitro-benzoesäure-Natriumsalz, welches ein konstitutionell ähnlicher, vorbekannter Wirkstoff gleicher Wirkungsrichtung ist.

Die erfindungsgemäßen substituierten Phenoxybenzoesäure-azolylalkylester sind durch die Formel (I) allgemein definiert. In dieser Formel stehen vorzugsweise

$X^1$ für Chlor,

$X^2$ für Wasserstoff oder Chlor,

$R^1$, $R^2$, $R^3$ und $R^4$ unabhängig voneinander für Wasserstoff oder Alkyl mit 1 oder 2 Kohlenstoffatomen,

m für 1 oder 2,

n für 0 oder 1 und

Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest, wobei jeder dieser Azolyl-Reste ein- oder mehrfach gleichartig oder verschieden substituiert sein kann durch Fluor, Chlor, Brom, Jod, Alkyl mit 1 bis 4 Kohlenstoffatomen, welches seinerseits gegebenenfalls durch Fluor, Chlor, Cyano, Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen und/oder Alkoxycarbonyl mit 1 bis 4 Kohlenstoffatomen in der Alkoxygruppe substituiert ist, Alkoxy mit 1 bis 4 Kohlenstoffatomen, Cyano, Carboxy, Alkoxycarbonyl mit 1 bis 4 Kohlenstoffatomen in der Alkoxygruppe, Alkylcarbonyl mit 1 bis 4 Kohlenstoffatomen in der Alkylgruppe und/oder Phenyl, welches seinerseits gegebenenfalls durch Halogen, Alkyl mit 1 bis 4 Kohlenstoffatomen, Trifluormethyl, Cyano, Nitro und/oder Alkoxy mit 1 bis 4 Kohlenstoffatomen substituiert ist.

Besonders bevorzugt sind diejenigen erfindungsgemäßen substituierten Phenoxybenzoesäure-azolylalkylester der Formel (I), in denen

$X^1$ für Chlor steht,

$X^2$ für Wasserstoff oder Chlor steht,

$R^1$ und $R^2$ unabhängig voneinander für Wasserstoff oder Methyl stehen,

$R^3$ und $R^4$ unabhängig voneinander für Wasserstoff oder Methyl stehen,

m für 1 oder 2 steht,

n für 0 oder 1 steht und

Az für einen über ein Ring-Stickstoffatom gebundenen Pyrazolyl-, Imidazolyl-, 1,2,4-Triazolyl-, 1,2,3-Triazolyl- oder 1,3,4-Triazolyl-Rest steht, wobei jeder dieser Azolyl-Reste ein-, zwei- oder dreifach, gleichartig oder verschieden substituiert sein kann durch Fluor, Chlor, Brom, Alkyl mit 1 oder 2 Kohlenstoffatomen, welches seinerseits gegebenenfalls durch Fluor, Chlor, Cyano, Alkoxy mit 1 oder 2 Kohlenstoffatomen, Alkylthio mit 1 oder 2 Kohlenstoffatomen, Alkylsulfinyl mit 1 oder 2 Kohlenstoffatomen, Alkylsulfonyl mit 1 oder 2 Kohlenstoffatomen und/oder Alkoxycarbonyl mit 1 oder 2 Kohlenstoffatomen in der Alkoxygruppe substituiert ist, Alkoxy mit 1 oder 2 Kohlenstoffatomen, Alkylthio mit 1 oder 2 Kohlenstoffatomen, Cyano, Carboxy,

Alkoxycarbonyl mit 1 oder 2 Kohlenstoffatomen in der Alkoxygruppe, Alkylcarbonyl mit 1 oder 2 Kohlenstoffatomen in der Alkylgruppe und/oder Phenyl, welches seinerseits gegebenenfalls durch Fluor, Chlor, Brom, Alkyl mit 1 oder 2 Kohlenstoffatomen, Trifluormethyl, Cyano, Nitro und/oder Alkoxy mit 1 oder 2 Kohlenstoffatomen substituiert ist.

Bevorzugt sind außerdem Säureadditions-Salze von substituierten Phenoxybenzoesäure-azolylalkylestern der Formel (I), in denen $X^1$,$X^2$,$R^1$,$R^2$,$R^3$,$R^4$,Az, m und n die vorzugsweise genannten Bedeutungen haben. Besonders bevorzugt sind dabei solche Säureadditions-Salze, die durch Addition von Halogenwasserstoffsäure, wie zum Beispiel Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie zum Beispiel Essigsäure, Maleinsäure, Bernsteinsäure, Fumarsäure, Weinsäure, Zitronensäure, Salicylsäure, Sorbinsäure, Milchsäure, sowie Sulfonsäuren, wie zum Beispiel p-Toluolsulfonsäure und 1,5-Naphthalindisulfonsäure entstehen.

Bevorzugt sind auch Metallsalz-Komplexe von substituierten Phenoxybenzoesäure-azolylalkylestern der Formel (I), in denen $X^1$,$X^2$,$R^1$,$R^2$,$R^3$,$R^4$,Az,m und n die vorzugsweise genannten Bedeutungen haben. Besonders bevorzugt sind dabei solche Metallsalz-Komplexe, die als Kationen Metalle der II. bis IV. Hauptgruppe oder der I. und II. oder IV. bis VIII. Nebengruppe des Periodensystems der Elemente enthalten, wobei Kupfer, Zink, Mangan, Magnesium, Zinn, Eisen und Nickel beispielhaft genannt seien.

Anionen dieser Metallsalz-Komplexe sind vorzugsweise solche, die sich von Halogenwasserstoffsäuren, insbesondere Chlorwasserstoffsäure und Bromwasserstoffsäure, ferner von Phosphorsäure, Salpetersäure und Schwefelsäure ableiten.

Bevorzugt sind schließlich auch quaternäre Alkylierungsmittel-Additionssalze von substituierten Phenoxybenzoesäure-azolylalkylestern der Formel (I), in denen $X^1$,$X^2$, $R^1$,$R^2$,$R^3$,$R^4$,Az,m und n die vorzugsweise genannten Bedeutungen haben. Besonders bevorzugt sind dabei solche quaternären Salze, die durch Addition von Alkylchloriden, -bromiden und -iodiden mit jeweils 1 bis 4 Kohlenstoffatomen bzw. von Alkylsulfaten mit 1 bis 4 Kohlenstoffatomen je Alkylgruppe entstehen.

Verwendet man 5-(2-Chlor-4-trifluormethyl-phenoxy)-2-nitro-benzoesäurechlorid und 1-(1-Hydroxy-ethyl)-imidazol als Ausgangsstoffe, so läßt sich der Verlauf des erfindungsgemäßen Verfahrens (a) durch das folgende Formelschema wiedergeben:

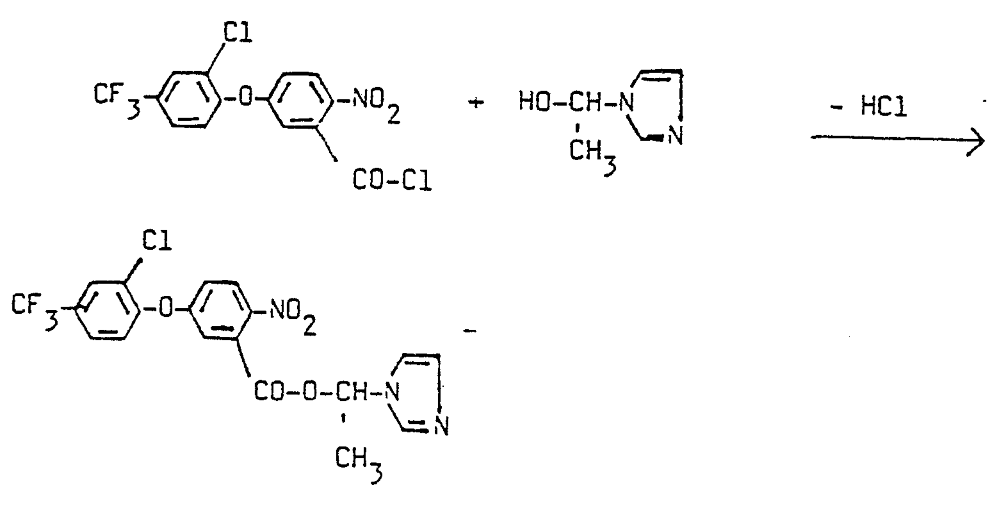

Verwendet man 3-(2,6-Dichlor-4-trifluormethyl-phenoxy)-benzoesäure-(1-pyrazolyl-methyl)-ester als Ausgangsstoff, so läßt sich der Verlauf des erfindungsgemäßen Verfahrens (b) durch das folgende Formelschema wiedergeben:

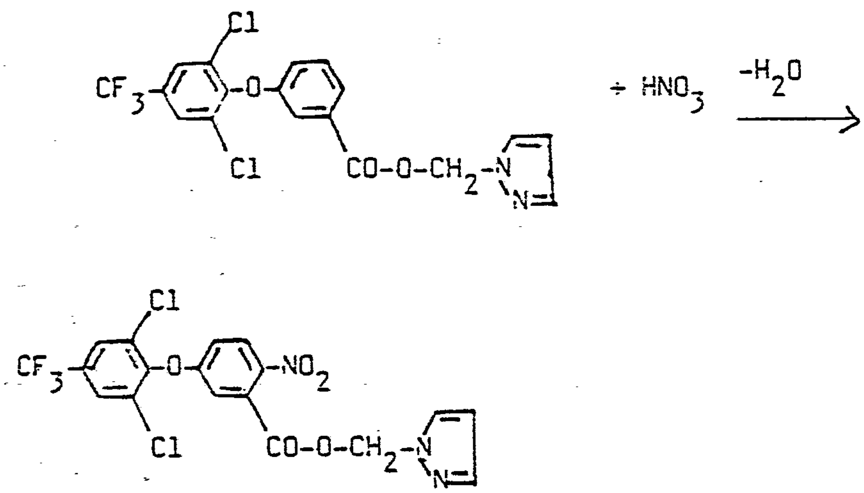


Verwendet man 2-Chlor-4-trifluormethylphenol und 5-Chlor-2-nitro-benzoesäure-(1,2,4-triazol-1-yl-methyl)-ester als Ausgangsstoffe, so läßt sich der Verlauf des erfindungsgemäßen Verfahrens (c) durch das folgende Formelschema wiedergeben:

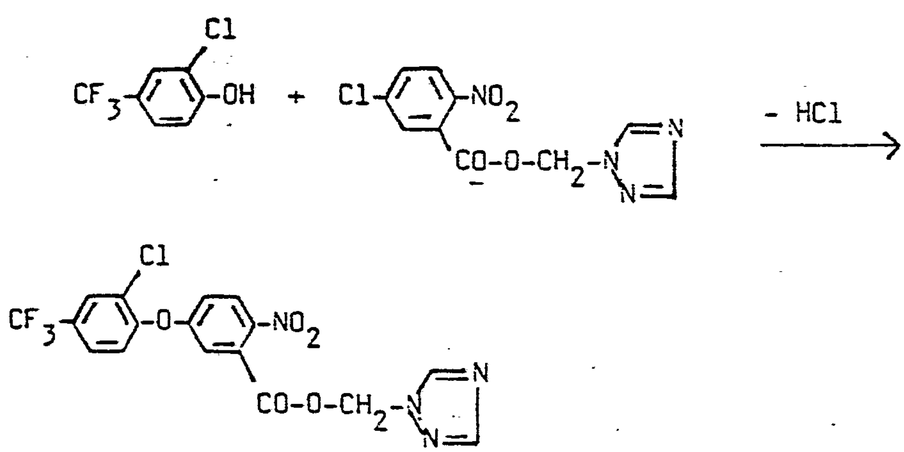

Die bei dem erfindungsgemäßen Verfahren (a) als Ausgangsstoffe benötigten substituierten Phenoxybenzoesäurechloride sind durch die Formel (II) allgemein definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele seien 5-(2-Chlor-4-trifluormethyl-phenoxy)-2-nitro-benzoesäurechlorid und 5-(2,6-Dichlor-4-trifluormethyl-phenoxy)-2-nitro-benzoesäurechlorid genannt.

Die Phenoxybenzoesäurechloride der Formel (II) sind zum Teil in der Literatur noch nicht beschrieben. Man erhält sie auf einfache Weise, indem man Phenoxybenzoesäuren der Formel

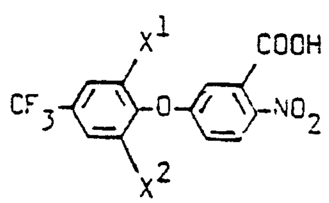

(VII)

in welcher

$X^1$ und $X^2$ die oben angegebene Bedeutung haben,

mit Chlorierungsmitteln, wie z.B. Thionylchlorid, gegebenenfalls unter Verwendung eines Katalysators, wie z.B. Dimethylformamid, und gegebenenfalls unter Verwendung eines Verdünnungsmittels, wie z.B. 1,2-Dichlorethan, bei Temperaturen zwischen 10 und 100°C, umsetzt und anschließend flüchtige Komponenten unter vermindertem Druck abdestilliert.

Die Phenoxybenzoesäuren der Formel (VII), - nämlich 5-(2-Chlor-4-trifluormethyl-phenoxy)-2-nitrobenzoesäure und 5-(2,6-Dichlor-4-trifluormethylphenoxy)-2-nitro-benzoesäure -, sind bereits bekannt (vgl. US-PS 3 928 416).

Die bei dem Verfahren (a) weiterhin als Ausgangsstoffe zu verwendenden Hydroxyalkylazole sind durch die Formel (III) definiert. In dieser Formel haben $R^1$, $R^2$, $R^3$, $R^4$, Az, m und n vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für Hydroxyalkylazole der Formel (III) seien im einzelnen genannt:
1-Hydroxymethyl-pyrazol, 1-(1-Hydroxyethyl)-pyrazol, 1-Hydroxymethylimidazol, 1-(1-Hydroxyethyl)-imidazol, 1-Hydroxymethyl-1,2,4-triazol, 1-(1-Hydroxyethyl)-1,2,4-triazol, 1-Hydroxymethyl-1,3,4-triazol, 1-(1-Hydroxyethyl)-1,3,4-triazol, 1-Hydroxymethyl-3,5-dimethylpyrazol, 1-Hydroxymethyl-2-methyl-imidazol, 1-Hydroxymethyl-3-methyl-pyrazol, 3-Chlor-1-hydroxymethyl-1,2,4-triazol, 4-Chlor-1-hydroxymethyl-pyrazol, 1-Hydroxy-



BAD ORIGINAL

methyl-4-methyl-pyrazol, 1-Hydroxymethyl-4-methoxy-pyrazol, 1-Hydroxymethyl-3-methylthio-pyrazol, 4-Chlor-1-hydroxymethyl-3,5-dimethylpyrazol, 1-(2-Hydroxyethyl)-pyrazol, 1-(2-Hydroxy-1,1-dimethyl-ethyl)-pyrazol und 1-(2-Hydroxyethyl)-1,2,4-triazol.

Die Verbindungen der Formel (III) sind bereits bekannt (vgl. DE-OS 2 835 157 und 2 835 158).

Die beim erfindungsgemäßen Verfahren (b) als Ausgangsstoffe benötigten Phenoxybenzoesäure-azolylalkylester sind durch die Formel (IV) allgemein definiert. In dieser Formel haben $X^1$,$X^2$,$R^1$,$R^2$,$R^3$,$R^4$,Az, m und n vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (IV) seien genannt: 3-(2-Chlor-4-trifluormethyl-phenoxy)-benzoesäure und 3-(2,6-Dichlor-4-trifluormethyl-phenoxy)-benzoesäure-pyrazol-1-yl-methylester, -1-(pyrazol-1-yl)-ethylester, -imidazol-1-yl-methylester, -1,2,4-triazol-1-yl-methylester, -1,3,4-triazol-1-yl-methylester, -(3,5-dimethylpyrazol-1-yl)-methylester, -(2-methyl-imidazol-1-yl)-methylester, -(3-methyl-pyrazol-1-yl)-methylester, -(3-chlor-1,2,4-triazol-1-yl)-methylester, -(4-chlor-pyrazol-1-yl)-methylester, -(4-methyl-pyrazol-1-yl)-methylester, -(4-methoxy-pyrazol-1-yl)-methylester, -(3-methylthio-pyrazol-1-yl)-methylester und -(4-chlor-3,5-dimethylpyrazol-1-yl)-methylester.

Die Verbindungen der Formel (IV) sind noch nicht in der



BAD ORIGINAL

Literatur beschrieben. Man erhält sie, wenn man Phenoxybenzoesäurechloride der Formel

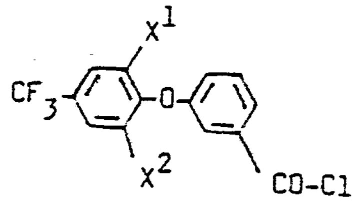

(VIII)

in welcher

$X^1$ und $X^2$ die oben angegebene Bedeutung haben,

mit Hydroxyalkyl-azolen der Formel

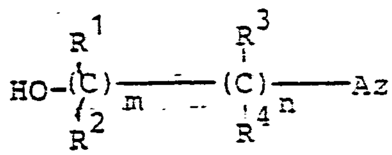

(III)

in welcher

$R^1$, $R^2$, $R^3$, $R^4$, Az, m und n die oben angegebene Bedeutung haben,

gegebenenfalls in Gegenwart eines Säureakzeptors und gegebenenfalls in Gegenwart eines Verdünnungsmittels umsetzt.

Als Beispiele für die Verbindungen der Formel (VIII) seien 3-(2-Chlor-4-trifluormethyl-phenoxy)-benzoesäurechlorid und 3-(2,6-Dichlor-4-trifluormethyl-phenoxy)-benzoesäurechlorid genannt.

Die Verbindungen der Formel (VIII) sind noch nicht in der Literatur beschrieben. Man erhält sie jedoch auf einfache Weise, wenn man Phenoxybenzoesäuren der Formel

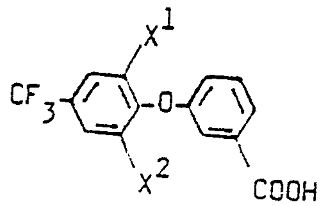

(IX) ,

in welcher

$X^1$ und $X^2$ die oben angegebene Bedeutung haben,

mit Chlorierungsmitteln, wie z.B. Thionylchlorid, gegebenenfalls unter Verwendung eines Katalysators, wie z.B. Dimethylformamid, und gegebenenfalls unter Verwendung eines Verdünnungsmittels, wie z.B. 1,2-Dichlorethan, bei Temperaturen zwischen 10 und 100°C, umsetzt und anschließend flüchtige Komponenten unter vermindertem Druck abdestilliert.

Die Phenoxybenzoesäuren der Formel (IX), nämlich 3-(2-Chlor-4-trifluormethyl-phenoxy)-benzoesäure und 3-(2,6-Dichlor-4-trifluormethyl-phenoxy)-benzoesäure, sind bekannt oder können nach im Prinzip bekannten Verfahren hergestellt werden (vgl. US-PS 4 031 131). So erhält man die Phenoxybenzoesäuren der Formel (IX) zum Beispiel dadurch, daß man Benzotrifluorid-Derivate der Formel



BAD ORIGINAL

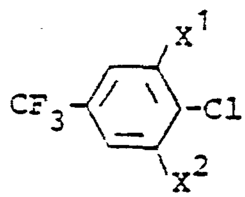

(X)

in welcher

$X^1$ und $X^2$ die oben angegebene Bedeutung haben,

mit 3-Hydroxybenzoesäure oder deren Salzen in Gegenwart eines Säurebindemittels, wie zum Beispiel Kaliumhydroxid oder Calciumhydroxid, sowie in Gegenwart eines Verdünnungsmittels wie zum Beispiel Methanol, bei Temperaturen zwischen 100 und 200°C umsetzt.

Bei dem obigen Verfahren zur Herstellung der Phenoxybenzoesäure-azolylalkylester der Formel (IV) entsprechen die Reaktionsbedingungen im einzelnen denjenigen des erfindungsgemäßen Verfahrens (a).

Die beim erfindungsgemäßen Verfahren (c) als Ausgangsstoffe zu verwendenden substituierten Phenole sind durch die Formel (V) allgemein definiert. In dieser Formel haben $X^1$ und $X^2$ vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele seien 2-Chlor-4-trifluormethylphenol und 2,6-Dichlor-4-trifluormethyl-phenol genannt.



BAD ORIGINAL

Die substituierten Phenole der Formel (V) sind bereits bekannt (vgl. DE-OS 2 016 624).

Die bei dem erfindungsgemäßen Verfahren (c) weiterhin als Ausgangsstoffe benötigten halogensubstituierten Benzoesäurederivate sind durch die Formel (VI) allgemein definiert. In dieser Formel haben $R^1$,$R^2$,$R^3$,$R^4$,Az, m und n vorzugsweise diejenigen Bedeutungen, die bereits im Zusammenhang mit der Beschreibung der erfindungsgemäßen Stoffe der Formel (I) vorzugsweise für diese Reste genannt wurden.

Als Beispiele für die Verbindungen der Formel (VI) seien genannt:
5-Brom-, 5-Chlor- und 5-Fluor-2-nitro-benzoesäure-pyrazol-1-yl-methylester, -1-(pyrazol-1-yl)-ethylester, -imidazol-1-yl-methylester, -1,2,4-triazol-1-yl-methylester, -1,3,4-triazol-1-yl-methylester, -(3,5-dimethylpyrazol-1-yl)-methylester, -(2-methyl-imidazol-1-yl)-methylester, -(3-methyl-pyrazol-1-yl)-methylester, -(3-chlor-1,2,4-triazol-1-yl)-methylester, -(4-chlor-pyrazol-1-yl)-methylester, -(4-methyl-pyrazol-1-yl)-methylester, -(4-methoxy-pyrazol-1-yl)-methylester, -(3-methylthio-pyrazol-1-yl)-methylester und -(4-chlor-3,5-dimethylpyrazol-1-yl)-methylester.

Die halogen-substituierten Benzoesäurederivate der Formel (VI) sind noch nicht literaturbekannt. Sie können jedoch nach üblichen Methoden hergestellt werden. Man



BAD ORIGINAL

erhält sie beispielsweise, wenn man 5-Halogen-2-nitrobenzoesäurechloride der Formel

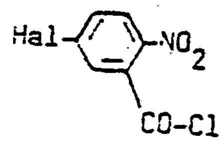

(XI)

in welcher

Hal für Fluor, Chlor oder Brom steht,

mit Hydroxyalkyl-azolen der Formel

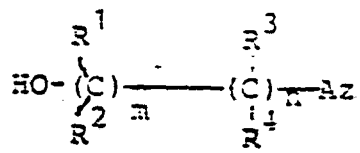

(III)

in welcher

$R^1$, $R^2$, $R^3$, $R^4$, Az, m und n die oben angegebene Bedeutung haben,

gegebenenfalls in Gegenwart eines Säureakzeptors und gegebenenfalls in Gegenwart eines Verdünnungsmittels umsetzt.

Bei dem obigen Verfahren entsprechen die Umsetzungsbedingungen im einzelnen denjenigen des erfindungsgemäßen Verfahrens (a).

Das erfindungsgemäße Verfahren (a) wird vorzugsweise in Gegenwart eines Verdünnungsmittels durchgeführt. Als derartige Verdünnungsmittel klmmen praktisch alle inerten organischen Verdünnungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methyl-ethyl-, Methyl-isopropyl- und Methyl-isobutylketon, außerdem Ester, wie Essigsäure-methylester und -ethylester, ferner Nitrile, wie z.B. Acetonitril und Propionitril, darüber hinaus Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Als Säureakzeptoren können bei dem erfindungsgemäßen Verfahren (a) alle üblichen Säurebindemittel verwendet werden. Hierzu gehören vorzugsweise Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperaturen können bei dem erfindungsgemäßen Verfahren (a) innerhalb eines größeren Bereichs variiert werden. Im allgemeinen arbeitet man bei Temperaturen zwischen -20 und 100°C, vorzugsweise zwischen 0 und 80°C. Das Verfahren wird im allgemeinen unter Normaldruck durchgeführt.

Zur Durchführung des erfindungsgemäßen Verfahrens (a) werden die Ausgangsstoffe der Formeln (II) und (III) in angenähert äquimolaren Mengen eingesetzt. Ein größerer Überschuß der einen oder anderen Komponente bringt keine größeren Vorteile. Die Umsetzung wird vorzugsweise in einem geeigneten Verdünnungsmittel in Gegenwart eines Säureakzeptors durchgeführt. Das Reaktionsgemisch wird mehrere Stunden bei der erforderlichen Temperatur gerührt. Die Aufarbeitung wird nach üblichen Methoden durchgeführt. Bei Raumtemperatur kristalline Produkte erhält man durch Verdünnen des Reaktionsgemisches mit Wasser und Absaugen.

Bei einer anderen Aufarbeitungsweise wird das Reaktionsgemisch mit Wasser und einem mit Wasser praktisch nicht mischbaren organischen Lösungsmittel, wie z.B. Toluol versetzt und geschüttelt. Die organische Phase wird abgetrennt, mit Wasser gewaschen, getrocknet, filtriert und eingeengt, wobei man das jeweilige Produkt der Formel (I) als Rückstand erhält.

Das erfindungsgemäße Verfahren (b) wird gegebenenfalls

unter Verwendung eines Verdünnungsmittels durchgeführt. Als Verdünnungsmittel kommen hierbei vorzugsweise halogenierte Kohlenwasserstoffe, wie z.B. Methylenchlorid, in Betracht.

Das erfindungsgemäße Verfahren (b) wird gegebenenfalls in Gegenwart eines Katalysators durchgeführt. Als Katalysator kommen vorzugsweise Protonensäuren, wie z.B. Schwefelsäure oder Essigsäure, in Betracht.

Die Reaktionstemperatur wird bei Verfahren (b) im allgemeinen zwischen -20 und +80°C, vorzugsweise zwischen 0 und 50°C gehalten. Verfahren (b) wird im allgemeinen bei Normaldruck durchgeführt.

Zur Durchführung des erfindungsgemäßen Verfahrens (b) setzt man auf 1 Mol Phenoxybenzoesäureazolylalkylester der Formel (IV) im allgemeinen 0,9 bis 2 Mol, vorzugsweise 1,0 bis 1,3 Mol Salpetersäure und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden, beispielsweise durch Eingießen des Reaktionsgemisches in Eiswasser, Absaugen und gegebenenfalls Umkristallisieren. Es ist auch möglich, so zu arbeiten, wie es oben für Verfahren (a) beschrieben wurde.



BAD ORIGINAL

Das erfindungsgemäße Verfahren (c) wird vorzugsweise in Gegenwart eines Verdünnungsmittels durchgeführt. Als Verdünnungsmittel kommen hierbei vorzugsweise alle diejenigen Solventien in Betracht, welche bereits im Zusammenhang mit der Beschreibung des erfindungsgemäßen Verfahrens (a) vorzugsweise genannt wurden.

Das erfindungsgemäße Verfahren (c) wird vorzugsweise in Gegenwart eines Säureakzeptors durchgeführt. Als Säureakzeptoren kommen hierbei vorzugsweise diejenigen Säurebindemittel in Betracht, welche bereits im Zusammenhang mit der Beschreibung des erfindungsgemäßen Verfahrens (a) vorzugsweise genannt wurden.

Die Reaktionstemperaturen können auch beim erfindungsgemäßen Verfahren (c) in einem größeren Bereich variiert werden. Im allgemeinen arbeitet man bei Temperaturen zwischen 0 und 100°C, vorzugsweise zwischen 10 und 80°C. Das erfindungsgemäße Verfahren (c) wird im allgemeinen bei Normaldruck durchgeführt.

Zur Durchführung des erfindungsgemäßen Verfahrens (c) werden die Ausgangsstoffe der Formeln (V) und (VI) in angenähert äquimolaren Mengen eingesetzt. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Die Aufarbeitung wird nach üblichen Methoden, beispielsweise wie oben für Verfahren (a) angegeben, durchgeführt.



BAD ORIGINAL

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren isoliert und gegebenenfalls durch Waschen mit einem inerten organischen Lösungsmittel gereinigt werden.

Zur Herstellung von Metallsalz-Komplexen von Verbindungen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Zur Herstellung von quaternären Alkylierungsmittel-Additionssalzen von Verbindungen der Formel (I) kommen vorzugsweise diejenigen Alkylchloride, -bromide, -iodide und Alkylsulfate in Betracht, die bereits im Zusammenhang mit der Beschreibung der erfindungsgemäßen Stoffe vorzugsweise als Alkylierungsmittel genannt wurden.

Die quaternären Alkylierungsmittel-Additionssalze der Verbindungen der Formel (I) können in einfacher Weise unter den für derartige Alkylierungen üblichen Bedingungen hergestellt werden. So erhält man die betreffenden quaternären Salze zum Beispiel dadurch, daß man eine Verbindung der Formel (I) in einem inerten organischen Solvens, wie zum Beispiel Acetonitril, löst und das Alkylierungsmittel hinzugibt. Die quaternären Salze, die kristallin anfallen, lassen sich nach üblichen Methoden isolieren. Im allgemeinen werden die Stoffe durch Abfiltrieren isoliert und gegebenenfalls durch Waschen mit einem inerten organischen Lösungsmittel gereinigt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen

Stoffe als totale oder selektive Herbizide wirken, hängt im wesentlichen von der angegebenen Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe sind besonders gut zur selektiven Unkrautbekämpfung in verschiedenen Kulturen, wie zum Beispiel Mais, Sojabohnen, Baumwolle, und Weizen, geeignet. Darüber hinaus können sie auch gut zur Entlaubung und Austrocknung der Blätter bei Baumwolle eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln..

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ester und Ether, Ketone, wie Aceton, Methylethylketon, Methylisobutylketon oder Cyclohexanon, stark polare Lösungsmittel wie Dimethylformamid und Dimethylsulfoxid, sowie Wasser.

Als feste Trägerstoffe kommen in Frage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate;
als feste Trägerstoffe für Granulate kommen in Frage:
z.B. gebrochene und fraktionierte natürliche Gesteine wie Calcit, Marmor, Bims, Sepiolith, Dolomit sowie



BAD ORIGINAL

synthetische Granulate aus anorganischen und organischen Mehlen sowie Granulate aus organischem Material wie Sägemehl, Kokosnußschalen, Maiskolben und Tabakstengel; als Emulgator- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Diese Formulierungen enthalten im allgemeinen zwischen 0,1 und 90 Gew.-% Wirkstoff, vorzugsweise zwischen 0,5 und 90 %.

Die erfindungsgemäß verwendbaren Wirkstoffe können als solche oder in ihren Formulierungen auch in Mischung

mit bekannten Herbiziden zur Unkrautbekämpfung Verwendung finden, wobei Fertigformulierungen oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dam Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken, sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die erfindungsgemäßen Wirkstoffe zeigen bei Nachlaufanwendung wachstumsregulierende Eigenschaften.

Die Herstellung und die Verwendung der erfindungsgemäßen Wirkstoffe gehen aus den nachfolgenden Beispielen hervor.

Herstellungsbeispiele

Beispiel 1

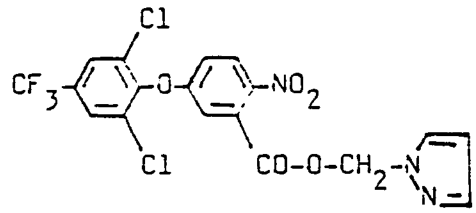

Eine Lösung von 20,7 g (0,05 Mol) 5-(2,6-Dichlor-4-trifluormethylphenoxy)-2-nitro-benzoesäurechlorid in 30 ml Acetonitril wird zu einer auf 0 bis 5°C gekühlten Mischung aus 5 g 1-Hydroxymethylpyrazol, 6 g Triethylamin und 70 ml Acetonitril gegeben. Das Gemisch wird über Nacht (ca. 15 Stunden) bei Raumtemperatur (ca. 20°C) gerührt. Dann wird mit Wasser verdünnt und mit Toluol extrahiert. Die organische Phase wird mit Wasser gewaschen, getrocknet, filtriert und eingeengt. Man erhält 19,3 g (81 % der Theorie) 5-(2,6-Dichlor-4-trifluormethyl-phenoxy)-2-nitro-benzoesäure-(1-pyrazol-methyl)-ester vom Schmelzpunkt 106°C als beigefarbenen kristallinen Rückstand.

Nach der im Beispiel 1 beschriebenen Methode oder auch nach den Verfahrensvarianten (b) und (c) werden die in den nachstehenden Tabellen formelmäßig aufgeführten Verbindungen hergestellt.

Tabelle 1

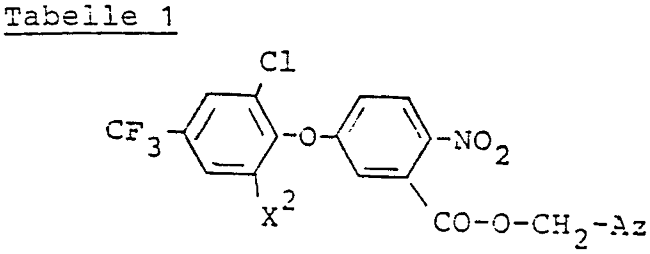

(Ia)



BAD ORIGINAL

| Beispiel Nr. | $X^2$ | Az | Schmelzpunkt (°C) Brechungsindex ($n_D^{20}$:) |
|---|---|---|---|
| 2 | H | 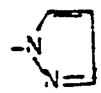 | 1,5638 |
| 3 | H | 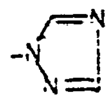 | |
| 4 | Cl | 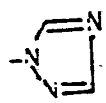 | |
| 5 | H | 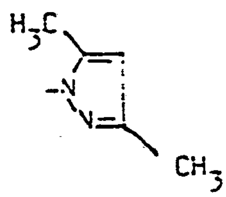 | |
| 6 | Cl | 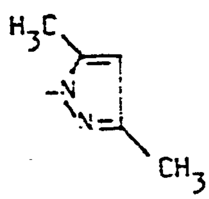 | |
| 7 | H | 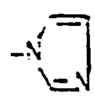 | |
| 8 | Cl | 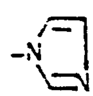 | |

Tabelle 1 (Fortsetzung)

| Beispiel Nr. | $X^2$ | Az | Schmelzpunkt (°C) Brechungsindex ($n_D^{20}$:) |
|---|---|---|---|
| 9 | H | 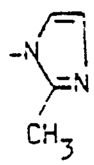 | |
| 10 | Cl | 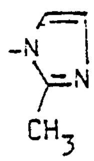 | |
| 11 | H | 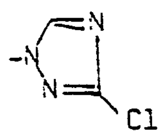 | |
| 12 | Cl | 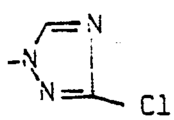 | |
| 13 | H | 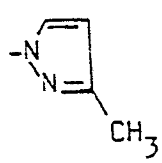 | |
| 14 | Cl | 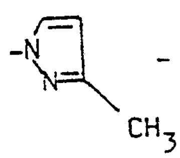 | |

Tabelle 1 (Fortsetzung)

| Beispiel Nr. | $X^2$ | Az | Schmelzpunkt (°C) Brechungsindex ($n_D^{20}$:) |
|---|---|---|---|
| 15 | H | 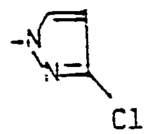 | |
| 16 | Cl | 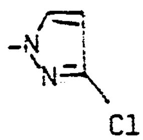 | |
| 17 | H | 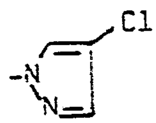 | |
| 18 | Cl | 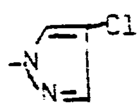 | |
| 19 | H | 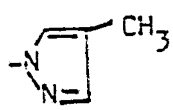 | |
| 20 | Cl | 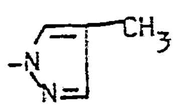 | |
| 21 | H | 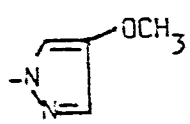 | |

Tabelle 1 (Fortsetzung)

| Beispiel Nr. | $X^2$ | Az | Schmelzpunkt (°C) Brechungsindex ($n_D^{20}$:) |
|---|---|---|---|
| 22 | Cl | 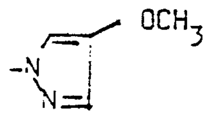 | |
| 23 | H | 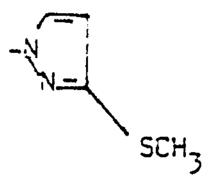 | |
| 24 | Cl | 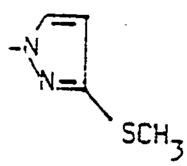 | |
| 25 | H | 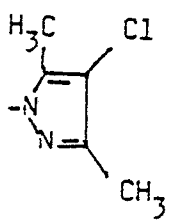 | |
| 26 | Cl | 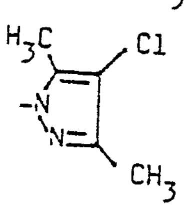 | |

Tabelle 2

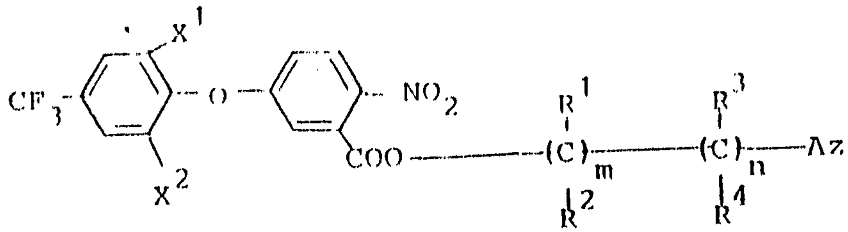 (I)

| Beispiel Nr. | $X^1$ | $X^2$ | $R^1$ | $R^2$ | $R^3$ | $R^4$ | m | n | Az | Schmelzpunkt [°C] bzw. Brechungsindex [$n_D^{20}$] |
|---|---|---|---|---|---|---|---|---|---|---|
| 27 | Cl | Cl | H | H | – | – | 2 | 0 | 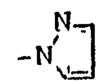 | 105°C |
| 28 | Cl | H | H | H | – | – | 2 | 0 | 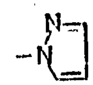 | 63°C |
| 29 | Cl | H | H | H | $CH_3$ | $CH_3$ | 1 | 1 | 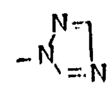 | 1,5479 |
| 30 | Cl | Cl | H | H | $CH_3$ | $CH_3$ | 1 | 1 | 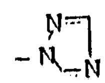 | |

In den folgenden Verwendungsbeispielen wird die nachstehend angegebene Verbindung als Vergleichssubstanz eingesetzt:

(A) = 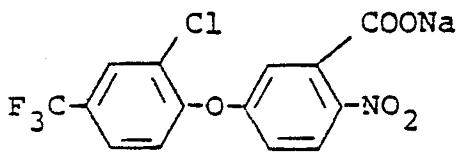

(bekannt aus DE-OS 2 311 638 bzw. US-PS 4 063 929)
5-(2-Chlor-4-trifluormethyl-phenoxy)-2-nitrobenzoesäure-Natriumsalz

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (1) und (2) eine bessere selektive herbizide Wirksamkeit als die Vergleichssubstanz (A).

Formulierungsbeispiel

Zur Herstellung eines Spritzpulvers werden

| | | |
|---|---|---|
| 50 | Gewichtsteile | Wirkstoff gemäß Beispiel 1, |
| 3 | " | Netzmittel auf Basis von Alkylarylsulfonat |
| 3 | " | Dispergiermittel auf Basis von Ligninsulfonat |
| 7 | " | Alkylarylsulfonat |
| 3 | " | Kondensationsprodukt aus Cyclohexanon, Formaldehyd und Natriumbisulfit |
| 1 | " | Ethylendiamin-tetraessigsäure-Dinatriumsalz |
| 33 | " | hochdisperse Kieselsäure |

in einem Lödige-Mischer intensiv gemischt und anschließend in einem Mikronizer 8" fein gemahlen. Das erhaltene Pulver wird vor der Ausbringung auf die Pflanzen in der jeweils gewünschten Menge mit Wasser vermischt.

Patentansprüche

1. Substituierte Phenoxybenzoesäure-azolylalkylester der Formel

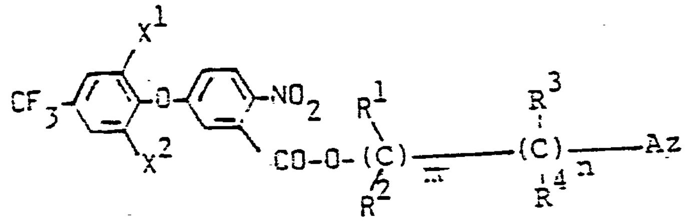

(I)

in welcher

$X^1$ und $X^2$ unabhängig voneinander für Wasserstoff oder Halogen stehen,

$R^1$, $R^2$, $R^3$, und $R^4$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salze, Metallsalz-Komplexe und quaternäre Alkylierungsmittel-Additionssalze.

2. Verfahren zur Herstellung von substituierten Phenoxybenzoesäure-azolylalkylestern der Formel

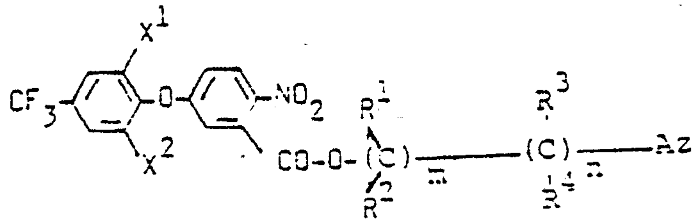

(I)

in welcher

$X^1$ und $X^2$ unabhängig voneinander für Wasserstoff oder Halogen stehen,

$R^1$,$R^2$,$R^3$ und $R^4$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

sowie deren Säureadditions-Salzen, Metallsalz-Komplexen und quaternären Alkylierungsmittel-Additionssalzen, dadurch gekennzeichnet, daß man



BAD ORIGINAL

a) substituierte Phenoxybenzoesäurechloride der Formel

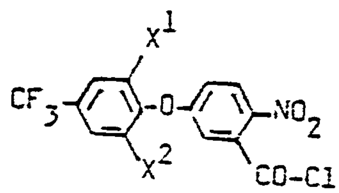

(II)

in welcher

$X^1$ und $X^2$ die oben angegebene Bedeutung haben,

mit Hydroxyalkyl-azolen der Formel

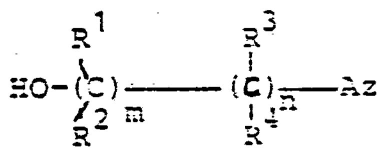

(III)

in welcher

$R^1$, $R^2$, $R^3$, $R^4$, Az, m und n die oben angegebene Bedeutung haben,

gegebenenfalls in Gegenwart eines Säureakzeptors und gegebenenfalls in Gegenwart eines Verdünnungsmittels umsetzt, oder

b) Phenoxybenzoesäure-azolylalkylester der Formel

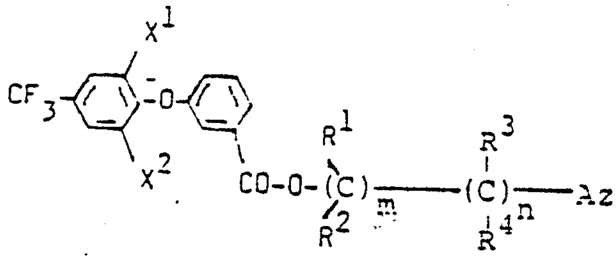

(IV)

in welcher

$X^1, X^2, R^1, R^2, R^3, R^4$, Az, m und n die oben angegebene Bedeutung haben,

mit Salpetersäure gegebenenfalls in Gegenwart eines Katalysators und gegebenenfalls in Gegenwart eines Verdünnungsmittels umsetzt, oder

c) substituierte Phenole der Formel

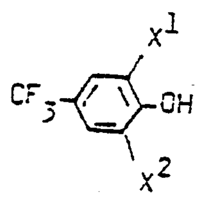

(V)

in welcher

$X^1$ und $X^2$ die oben angegebene Bedeutung haben,

mit Halogen-substituierten Benzoesäurederivaten der Formel

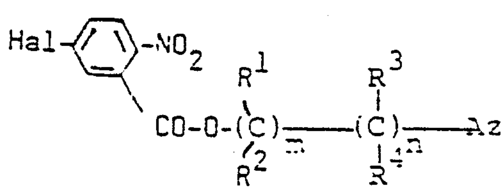

(VI)

in welcher

$R^1$,$R^2$,$R^3$,$R^4$,Az, m und n die oben angegebene Bedeutung haben und

Hal für Fluor, Chlor oder Brom steht,

gegebenenfalls in Gegenwart eines Säureakzeptors und gegebenenfalls in Gegenwart eines Verdünnungsmittels umsetzt,

und gegebenenfalls anschließend an die so erhaltenen substituierten Phenoxybenzoesäureazolylalkylester der Formel (I) eine Säure, ein Metallsalz oder ein Alkylierungsmittel addiert.

3. Herbizide Mittel, gekennzeichnet durch einen Gehalt an mindestens einem substituierten Phenoxybenzoesäure-azolylalkylester der Formel (I) bzw. einem Säureadditions-Salz, Metallsalz-Komplex oder quaternärem Alkylierungsmittel-Additionssalz von einem substituiertem Phenoxybenzoesäure-azolylalkylester der Formel (I).

4. Verwendung von substituierten Phenoxybenzoesäure-azolylalkylestern der Formel (I) bzw. von deren Säureadditions-Salzen, Metallsalz-Komplexen und quaternären Alkylierungsmittel-Additionssalzen zur Bekämpfung von Unkräutern.

5. Phenoxybenzoesäure-azolylalkylester der Formel

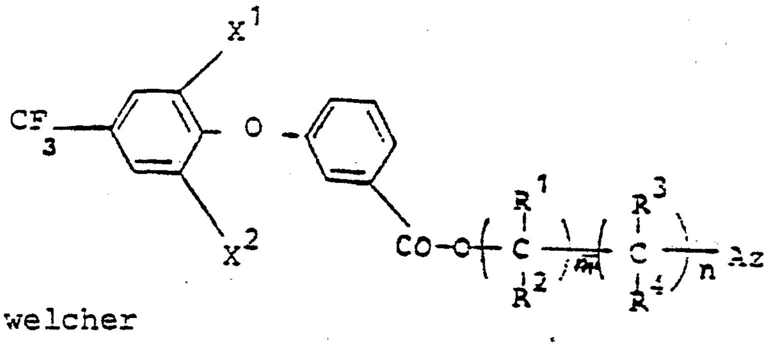

(IV)

in welcher

$X^1$ und $X^2$ unabhängig voneinander für Wasserstoff oder Halogen stehen,

$R^1$,$R^2$,$R^3$ und $R^4$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht.

6. Verfahren zur Herstellung von Phenoxybenzoesäure-azolylalkylestern der Formel

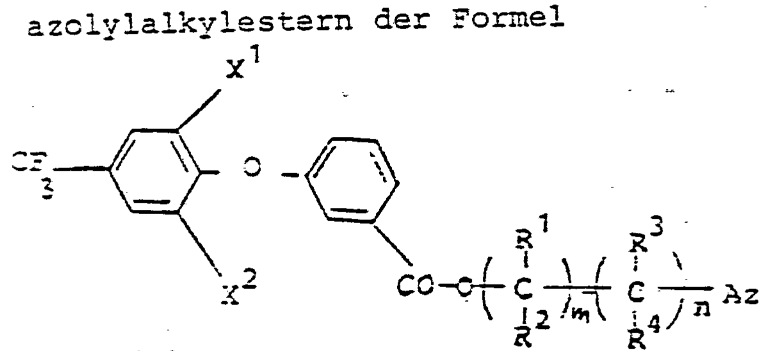

(IV)

in welcher

$X^1$ und $X^2$ unabhängig voneinander für Wasserstoff oder Halogen stehen,

$R^1$, $R^2$, $R^3$ und $R^4$ unabhängig voneinander für Wasserstoff oder Alkyl mit 1 bis 4 Kohlenstoffatomen stehen,

m für 1 oder 2 steht,

n für 0 oder 1 steht und

Az für einen gegebenenfalls substituierten Azolylrest steht,

dadurch gekennzeichnet, daß man Phenoxy-benzoesäurechloride der Formel

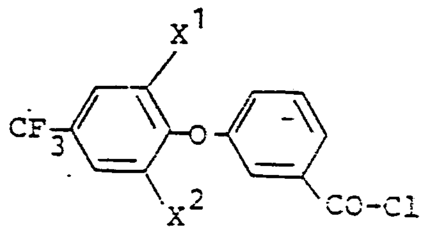

(VIII)

in welcher

$X^1$ und $X^2$ die oben angegebene Bedeutung haben,

mit Hydroxyalkyl-azolen der Formel

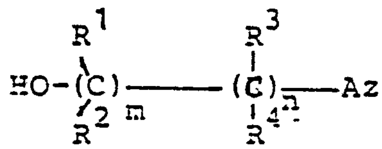

(III)

in welcher

$R^1$, $R^2$, $R^3$, $R^4$, Az, m und n die oben angegebene Bedeutung haben,

gegebenenfalls in Gegenwart eines Säureakzeptors und gegebenenfalls in Gegenwart eines Verdünnungsmittels umsetzt.

7. Halogensubstituierte Benzoesäurederivate der Formel

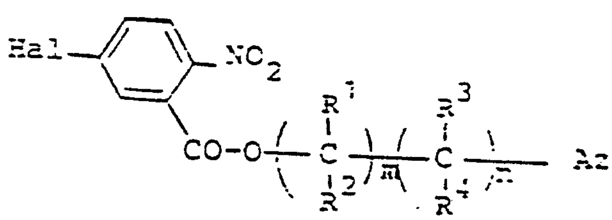

(VI)

in welcher

$R^1$, $R^2$, $R^3$ und $R^4$ unabhängig voneinander für Wasserstoff oder Alkyl mit 1 bis 4 Kohlenstoffatomen stehen,

m für 1 oder 2 steht,

n für 0 oder 1 steht,

Az für einen gegegebenenfalls substituierten Azolylrest steht und

Hal für Fluor, Chlor oder Brom steht.

8. Verfahren zur Herstellung von halogen-substituierten Benzoesäurederivaten der Formel

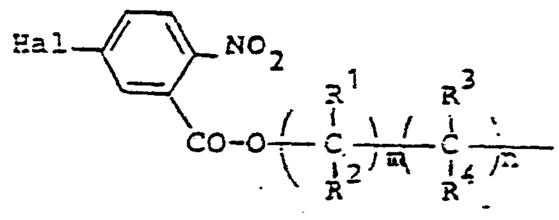

(VI)

in welcher

$R^1$ und $R^2$ unabhängig voneinander für Wasserstoff oder Alkyl mit 1 bis 4 Kohlenstoffatomen stehen,

m für 1 oder 2 steht,

n für 0 oder 1 steht,

Az für einen gegebenenfalls substituierten Azolylrest steht und

Hal für Fluor, Chlor oder Brom steht,

dadurch gekennzeichnet, daß man 5-Halogen-2-nitrobenzoesäurechloride der Formel

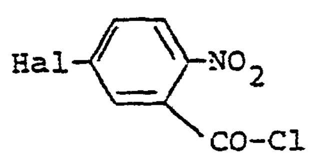

(XI)

in welcher

Hal die oben angegebene Bedeutung hat,

mit Hydroxyalkyl-azolen der Formel

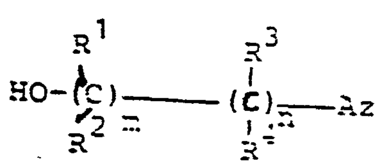

(III)

in welcher
$R^1$, $R^2$, $R^3$, $R^4$, Az, m und n die oben angegebenen Bedeutung haben,

gegebenenfalls in Gegenwart eines Säureakzeptors und gegebenenfalls in Gegenwart eines Verdünnungsmittels umsetzt.

9. Phenoxybenzoesäurechloride der Formel

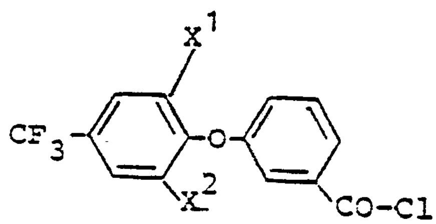

(VIII)

in welcher
$X^1$ und $X^2$ unabhängig voneinander für Wasserstoff oder Halogen stehen.

10. Verfahren zur Herstellung von Phenoxy-benzoesäurechloriden der Formel

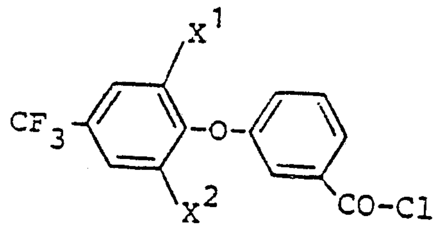

(VIII)

in welcher
$X^1$ und $X^2$ unabhängig voneinander für Wasserstoff oder Halogen stehen,

dadurch gekennzeichnet, daß man Phenoxybenzoesäuren der Formel

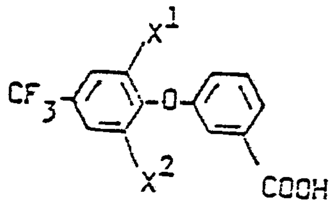

(IX)

in welcher

$X^1$ und $X^2$ die oben angegebene Bedeutung haben,

mit Chlorierungsmitteln gegebenenfalls in Gegenwart eines Katalysators sowie gegebenenfalls in Gegenwart eines Verdünnungsmittels umsetzt.